# EUROPEAN PATENT SPECIFICATION

(11) **EP 1 223 998 B1**
(45) Date of publication and mention of the grant of the patent: **10.10.2007**
(21) Application number: 00962261.4
(22) Date of filing: 29.09.2000
(51) Int. Cl.: A61M 5/172

(54) **AIR SHOT MECHANISM FOR ELECTRONIC INJECTION DEVICES**
ELEKTRONISCHE LUFTINJEKTIONEN
MECANISME DE PREPARATION DE DOSE DESTINE A DES DISPOSITIFS D'INJECTION ELECTRONIQUES

(30) Priority: 12.10.1999 DK 145199; 03.12.1999 DK 173299
(43) Date of publication of application: 24.07.2002
(73) Proprietor: NOVO NORDISK A/S, 2880 Bagsvaerd (DK)
(72) Inventor: MUNK, Jens, DK-3660 Stenlöse (DK); AASMUL, Sören, DK-2840 Holte (DK); LJUNGGREEN, Henrik, DK-2850 Ballerup (DK); CHRISTENSEN, Lars, Hofmann, DK-4040 Jyllinge (DK)
(86) International application number: PCT/DK2000/000538
(87) International publication number: WO 2001/026710

(56) References cited:
- WO-A-98/01168
- WO-A-98/10814

## Description

The present invention concern an electronic injection device comprising the features as defined in the preamble of claim 1. Such a device is known from WO-A-58/01168.

It is attractive to set the dose electronically and show the set dose on an electronic display, e.g. a LCD display, as the size of the digits displayed can be made arbitrarily large without being dependent on the distances possible mechanical dose setting parts are moved by the setting of the dose.

From US 5 928 201 is known a device by which a dose is set by operating a pair of dose setting buttons, one increasing the set dose step by step and another decreasing the dose step by step when operated. The set dose is stored in a circuit which controls a display to show the set dose and a motor drives a mechanical dose setting device to carefully set a mechanical dose setting device to the dose shown in the display. The injection is performed manually to allow the user to inject the dose with the rate he finds appropriate.

It may be attractive to let the injection be performed electro-mechanically too as then the demand to the users finger strength can be reduced to the force needed to press down an electronic switch button. Further the mechanical impact on the device is independent on the strength of the user so it is avoided that some users can hardly operate the device whereas other will break it.

Devices wherein the doses are electronically represented also may contain circuitry wherein historical data are stored so it is possible to create a picture of the way the user has followed his treatment and prospective doses and time intervals can be planned on the basis of the stored data. At least it is appropriate to be able to store the size of the latest injected dose and the time passed since this injection.

In an electronic injection device of the kind described above only a minimum of operation buttons are needed, e.g. a counting up dose set button allowing a stepwise increase of the set dose, a counting down dose set button allowing a stepwise decrease of an erroneously too high set dose, and an injection button by which either the dose is manually injected or the electronic circuit is ordered to energise a motor to inject the set dose.

By injecting medicine from an ampoule it is necessary to ensure that no air is left in the ampoule when the injection is performed. For this purpose a so-called air-shot is performed. An air shot may be performed by setting a small dose, e.g. one international unit of the medicine to be injected, and make this small dose be pressed out from the ampoule holding the device with the needle pointing vertically upward. This procedure can be repeated until it is seen that liquid is sprayed out through the needle where after the dose to be injected can be set, the needle can be inserted into the skin of the user, and an amount of medicine corresponding to the set dose can be pressed out and injected through the needle.

However, this repetitive use of the dose-setting device is not wanted. If the device is provided with a memory which stores historical data for the purpose of controlling the course of a treatment it is necessary to register which of the doses have been air shot doses and which doses has actually been injected. This problem could be overcome by providing a special air shot button as it is known from syringes by which the injection is performed manually, but this will be on the account of one more button whereby the possibilities for erroneous operation is increased.

An objective of the invention is to provide an injection device by which repetitive air shots can be performed without adding extra operation buttons to the device and without information of the air-shots are stored as a part of the operation history of the device.

This is obtained by an electronic injection device as described in the opening paragraph of this application which device is according to the invention characterised in the features defined in the characterising portion of claim 1.

This means that when the device is taken into use but no dose has been set the electronic circuit will work in a mode making it control either a dose setting motor to set a small air shot dose which can be injected by pressing the injection button, or control a motor to press out an air shot dose when the injection button is operated.

The injection button may now be operated one or more times while the device is held with the needle pointing upward until it is seen that liquid and not air is pressed out through the needle.

To ensure that the device is held in the correct position with the needle pointing upward a position indicator may be provided which gives of a signal to the electronic circuit when the device is held in the correct position for an air shot. The signal, which can be an opening or a closing of a switch, can be made a condition that has to be fulfilled before the electric circuit energises the motor to prepare or perform an air shot. This way waste of medicament, due to the fact that the device is held in a position in which air in the ampoule cannot reach the needle, is avoided.

The dose setting may be electro-mechanically realised by means of an electric motor which is controlled by the electronic circuit to lift up the injection button from the housing a distance corresponding to the set dose and the injection may be performed manually by pressing home the injection button. The signal that indicates that a set dose has been injected may be a switch which is actuated when the injection button is pressed. The electronic circuit will then prepare a new air shot each time a home pressing of the injection button is performed and the circuit will remain in the air shot mode.

In devices wherein the injection is performed electro-mechanically by a motor controlled by the electronic circuit, actuation of the injection button may make a motor electro-mechanically press out a dose from the ampoule, the motor being controlled by the electronic circuit to press out an automatically set small air shot dose when the circuit is working in its air shot mode, and a dose set by operation of the dose setting means when the circuit is working in its dose injection mode.

At the very moment when the dose setting member is operated the working mode of the electronic circuit is changed to an injection mode in which operation of the injection button will result in the injection of a set dose either by directly pressing out a dose corresponding to the distance the injection button has been elevated over the housing by the dose setting motor controlled by the electronic circuit, or by controlling an injection motor to inject the dose set by the operation of the dose setting member, and the circuit will remain in this mode until the injection button has been pressed.

If it is wanted to ensure that at least one air shot is performed in advance of an injection the circuit may be so designed that it cannot receive a signal from the dose setting means until at least one air shot dose has been administered.

According to a preferred embodiment of the invention the electronic circuit may comprise a memory wherein historical information of injected doses and the time for their injection is stored. As the electronic circuit according to its two working modes can discriminate between air shots and injection of doses set by the user, these historical data can be rid of disturbing air shot data.

The electronic circuit may further be provided with a memory wherein the size of all air shots and injections are summed and subtracted from the size of the total content of a new ampoule to leave the memory with an information of the size of the remaining amount of medicine in the ampoule.

The set dose currently is compared with the remaining amount of medicine in the ampoule to block for setting of a dose exceeding this remaining amount.

In the following the invention i described in further details with references to the drawing in which
**Figure 1** shows a block diagram of the electronic components of an embodiment of an injection device according to the invention,
Figure 2 shows a block diagram of the electronic components of another embodiment of an injection device according to the invention, and
Figure 3 schematically shows an injection device according to the invention.

In an injection device wherein the dose is injected by means of a motor which when energised presses out medicine from the ampoule an electronic circuit 1 is connected to a battery 2 which supplies the power needed for the operation of the circuit. Further the battery 2 supplies an injection motor 3 which power supply is controlled by the circuit 1. To the circuit 1 is coupled a set of dose setting buttons 4 and 5, one 4 marked with a "+" and which provides a stepwise increase of the set dose as long as it is pressed, and another 5 marked with a "-" and which provides a stepwise decrease of a set dose as long as it is pressed. During the setting of a dose the size of this set dose can be read on a display 6 coupled to electronic circuit 1. The dose-increasing button 4 is maintained in pressed condition until the display has counted up to the wanted dose, if the wanted dose is exceeded the button 5 is pressed until the display has counted down to the wanted dose. After the dose has been set the set dose is injected by pressing an injection button 7, which makes the circuit energise the motor to drive a mechanical injection mechanism to press out the set dose from the ampoule. A data connection 8 from the motor to the electronic circuit provides a feed back to the circuit of the dose injected and when this dose reaches the set dose the energising of the motor 3 is stopped.

The dose setting is here described as being performed by buttons 4 and 5. Rotating a dose setting wheel or drum whereby a counting up is performed when this wheel is rotated in one direction and a counting down is performed when the wheel is rotated in the opposite direction may alternatively perform the dose setting.

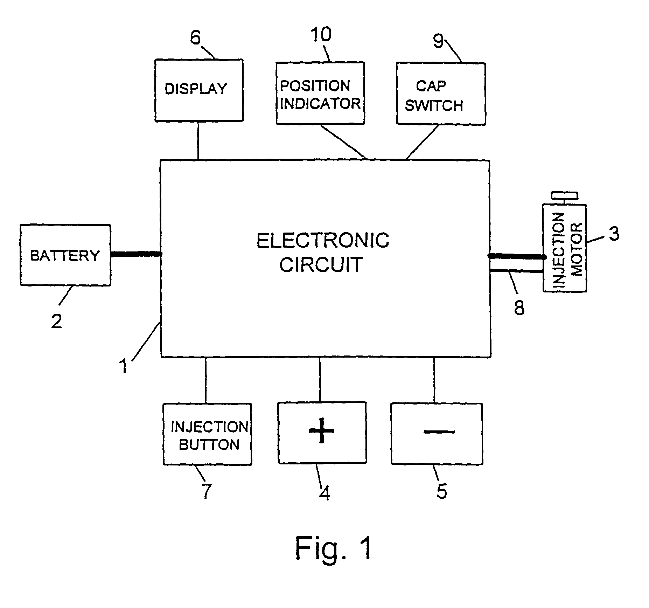

From WO 9733638 it is known to provide a device with a protective cap protecting the needle when the device is not in use. Further a switch is provided which is operated by the cap so that the position of the switch indicates whether the cap is on or not. In the device in figure 1 as witch 9 is coupled to the circuit 1 to bring information of the on or off condition of the cap to this circuit. To save battery power consuming parts of the circuit is turned off when the cap is on. This does not go for possible watch and memory functions which need continuous energising. When the cap is taken off a signal is sent to the circuit 1 which is then coupled to work in an air shot mode in which an activation of the injection button 7 will make the circuit energise the motor to make the device administer a pre-set small amount from the ampoule, e. g. an amount corresponding to one international unit of the medicine in the ampoule. Such an administration will be performed each time the injection button 7 is activated until a dose setting is started. As soon as the circuit receives a signal from the dose setting buttons 4 or 5 indicating that a dose is being set the working mode of the circuit 1 changes to a dose injection mode in which the motor is controlled to administer the set dose when the injection button 7 is pressed. When the set dose has been administered by pressing of the button 7 the circuit will revert to its air shot mode but if a new dose is set after a first dose has been injected the circuit quickly reverts to its dose injection mode until the button 7 is pressed again.

A switch connected to the injection button 7 is actuated each time the injection button is operated. When this switch is actuated it gives of a signal to the circuit, which signal is taken as an indication of the fact that the injection button has been operated.

When the circuit receives this signal it is coupled to work in an air shot mode in which an activation of the injection button 7 will make the circuit energise the motor to make the device administer a pre-set small amount from the ampoule, e. g. an amount corresponding to one international unit of the medicine in the ampoule. Such an administration will be performed each time the injection button 7 is activated until a dose setting is started. As soon as the circuit receives a signal from the dose setting buttons 4 or 5 indicating that a dose is being set the working mode of the circuit 1 changes to a dose injection mode in which the motor is controlled to administrate the set dose when the injection button 7 is pressed. When the button 7 has been pressed to inject the set dose the injection button switch gives of a signal making the circuit change to the air shot mode but as soon as the dose setting buttons are operated again the circuit will change to the dose injection mode.

During the air shots the device must be held with the needle pointing upward so that possible air in the ampoule lies as a bubble adjacent to the needle tip projecting into the ampoule. To avoid that medicine is wasted due to the fact that the device is not held in the correct vertical position a direction indicator 10 may be include in the device which indicator provides a signal which informs the circuit whether the position of the device is appropriate for an air shot.

Said signal can control the circuit to only energise the motor to perform an air shot when the device is in the correct position for such an air shot. When the circuit works in its dose-injecting mode the position will have no influence on the energising of the motor 3.

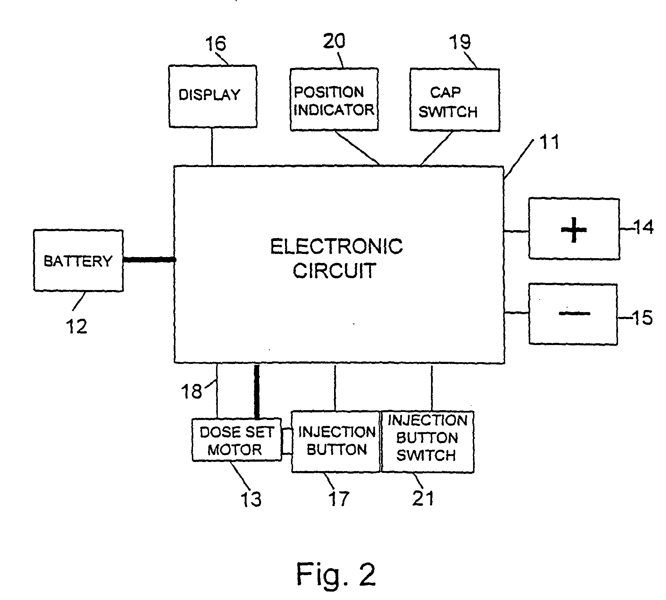

in a syringe according to figure 2, wherein an injection button 17 is elevated over the housing of the device a distance corresponding to a set dose by means of a motor 13, the power necessary for the running of the device is delivered by a battery 12 which energises an electronic circuit 11 which function much like the circuit 1 in figure 1. A dose is set by the dose setting buttons 14 and 15 which controls a counting up and down of a set dose size in the circuit 11. The set dose is shown in a display 16 and the motor 13 performs a mechanical setting of the dose by elevating the injection button 17 a distance corresponding to the set dose as the circuit controls the motor 13 to make it perform the elevation needed. A feed back 18 from the motor to the circuit enables this circuit to decide when the injection button has been elevated a distance corresponding to the dose read into the circuit by the operation of the dose setting buttons 14 and 15.

In the device described in figure 2 the air shot is obtained by the fact that the motor automatically elevates the injection button 17 a distance corresponding to an air shot dose as long as the circuit works in its air shot mode what it does after each pressing of the injection button 17. When one of the dose setting buttons 14 or 15 is operated the circuit 11 shifts to a dose injection mode and controls the motor 13 to elevate the injection button 17 a distance corresponding to the set dose. When the injection button is pressed the set dose is injected and the injection button sends a signal to the circuit 11 to make this circuit revert to the air-shot mode.

The device according to figure 2 is further provided with a position indicator 20 which gives off a signal to the electronic circuit which will only allow setting of an air shot dose when the device is held in a position vertical position with the needle pointing upward. The air shot function may be repeated as long as the dose setting buttons 14 or 15 are not operated. The actuation of the injection button 21 switch sends a signal to the electronic circuit making this circuit attain the air shot mode in which it controls the injection button to be elevated again after an air shot until a signal from one of the dose setting buttons 14 or 15 makes said electronic circuit shift to its dose injection mode.

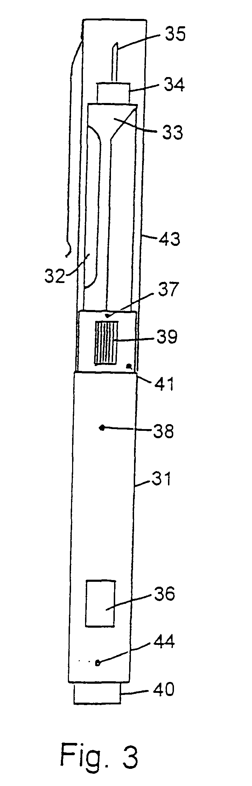

Figure 3 shows schematically an injection device comprising a housing 31 in which an ampoule holder 33 holds an ampoule 32. A needle hub 34 with an injection needle 35 is mounted at the distal end of the ampoule so that a not shown rear needle is penetrating the sealing of the ampoule. A dose-setting wheel 39 can be rotated in one direction to count up a dose or in the opposite direction to count down a too large set dose. Turning of the dose setting wheel 39 is by a signal generator or switch 37 reported to a not shown electronic circuit in the housing and the set dose is shown in a display 36. An injection button 40 is provided the operation of which will result in administration of a dose, which dose depending on the operation mode of the electronic circuit is a fixed small air shot dose or the dose shown in the display 36.

When a cap 43 protecting the needle 35 is removed and a switch 41 reports to the electronic circuit that the device is going to be taken into use and the electric interfaces which has been shut off during the storing of the device are energised.

Depending on the embodiment of the injection device the injection button 40 can operate an electric switch that activates the electronic circuit to energise a motor, which moves a piston into the ampoule to press out some of the content of this ampoule through the needle 35. In another embodiment of the injection device the button is mechanically lifted up from the end of the housing 31 a distance corresponding to the size of a set dose. In this embodiment the button is automatically elevated a distance corresponding to an air shot when the cap 43 is removed and an air shot is administered by pressing the button 40 home. As it may be necessary to repeat the air shot procedure, a switch 44 is provided which reports to the electronic circuit that the button 40 has been pressed home. As the circuit attains its air shot mode when it receives this signal, the button is again automatically elevated to a position corresponding to an air shot whereas it is elevated a distance corresponding to the dose set by the dose setting wheel 39 when this wheel is operated and the circuit is shifted to its injection mode.

The dose setting device is described as a dose setting wheel but it may as well be a count up button and a count down button without departing form the scope of the invention.

## Claims

1. An electronic injection device comprising a housing (31), dose setting means (4, 5/14, 15), an injection button (7, 17) and an electronic circus (1, 11), said electronic circuit is coupled to said dose setting means and said electronic circuit is adapted to read a dose size corresponding to the operation of the dose setting means, a medicine containing ampoule (32) in connection to a needle (35) mounted at the distal end of the ampoule, the device is adapted to press the medicine from the ampoule out through the needle when the injection button is operated, a display (6, 16, 36) driven by the electronic circuit adapted to show the dose set by operation of the dose setting means,
**characterized in that**, the electronic circuit is designed to work in two alternative modes:
an air shot mode in which it controls an automatic pre-setting of an air shot dose to be pressed from the ampoule out through the needle when the injection button is actuated,
and a dose injection mode in which a dose set by operation of the dose setting means is injected by operation of the injection button,
where said circuit is in the air shot mode when the device is taken into use but no dose has been set,
said circuit shifts to the dose injection mode when the dose setting means has been operated,
and said circuit returns to the air shot mode when the circuit receives a signal indicating that a set dose has been injected.

2. An electronic injection device according to claim 1, **characterised in that** a signal form a switch indicating that a protection cap (43) is mounted is taken as indicating that a set dose has been injected.

3. An electronic injection device according to claim 1, **characterised in that** a signal form a switch indicating that the injection button has been pressed is taken as indicating that a set dose has been injected.

4. An electronic injection device according to anyone of the preceding claims, **characterised in that** a position indicator is provided which controls the circuit to only perform said administration of an air shot dose when the position indicator indicates that the device is held vertically with the needle pointing upward.

5. An electronic injection device according to claim 1, **characterised in that** the dose setting is electro-mechanically realised by means of an electric motor (3, 13) which is controlled by the electronic circuit to lift up the injection button from the housing a distance corresponding to the set dose and the injection is performed manually by pressing home the injection button.

6. An electronic injection device according to claim 1, **characterised in that** actuation of the injection button makes a motor electro-mechanically press out a dose from the ampoule, the motor being controlled by the electronic circuit to press out an automatically set air shot dose when the circuit is working in its air shot mode and a dose set by operation of the dose setting means when the circuit is working in its dose injection mode.

7. an electronic injection device according to anyone of the preceding claims, **characterised in that** the circuit is so designed that it cannot receive a signal from the dose setting means until at least one air shot dose has been administered.

8. An electronic injection device according to anyone of the preceding claims, **characterised in that** the circuit comprises a memory wherein historical information of injected doses and the time for their injection are stored.

9. An electronic injection device according to anyone of the preceding claims, **characterised in that** the circuit comprises a memory wherein the size of all air shots and injections are summed and subtracted from the size of the total content of a new ampoule to leave the memory with an information of the size of the remaining amount of medicine in the ampoule.

10. An electronic injection device according to anyone of the preceding claims, **characterised in that** the set dose currently is compared with the remaining amount of medicine in the ampoule to enable blocking for setting of a dose exceeding this remaining amount.

## Patentansprüche

1. Elektronische Injektionsvorrichtung, umfassend ein Gehäuse (31), Dosiseinstellungsmittel (4, 5/14, 15), einen Injektionsknopf (7, 17) und eine elektronische Schaltung (1, 11), wobei die elektronische Schaltung an das Dosiseinstellungsmittel gekoppelt ist und die elektronische Schaltung zum Lesen einer Dosisgröße je nach Betrieb des Dosiseinstellungsmittels angepasst ist, eine ein Medikament enthaltende Ampulle (32) in Verbindung mit einer an das entfernte Ende der Ampulle befestigten Nadel (35), wobei die Vorrichtung zum Drücken des Medikaments aus der Ampulle heraus durch die Nadel beim Betätigen des Injektionsknopfs angepasst ist, eine durch die elek-tronische Schaltung angetriebene Anzeige (6, 16, 36), die zum Anzeigen der durch den Betrieb des Dosiseinstellungsmittels eingestellten Dosis angepasst ist,
**dadurch gekennzeichnet, dass** die elektronische Schaltung zum Arbeiten in zwei alternativen Modi gestaltet ist:
einem Luftschussmodus, in dem sie eine automatische Voreinstellung einer aus der Ampulle heraus durch die Nadel zu drückenden Luftschussdosis beim Betätigen des Injektionsknopfes steuert,
und einen Dosisinjektionsmodus, in dem eine durch Betrieb des Dosiseinstellungsmittels eingestellte Dosis durch Betrieb des Injektionsknopfs injiziert wird,
wobei die Schaltung im Luftschussmodus vorliegt, wenn die Vorrichtung in Betrieb genommen wird, jedoch keine Dosis eingestellt wurde,
sich die Schaltung zum Dosisinjektionsmodus verschiebt, wenn das Dosiseinstellungsmittel betätigt worden ist,
und die Schaltung zum Luftschussmodus zurückkehrt, wenn die Schaltung ein Signal empfängt, das anzeigt, dass eine eingestellte Dosis injiziert wurde.

2. Elektronische Injektionsvorrichtung nach Anspruch 1, **dadurch gekennzeichnet, dass** ein Signal von einem Schalter, das anzeigt, dass eine Schutzkappe (43) befestigt ist, als Anzeige dafür verwendet wird, dass eine eingestellte Dosis injiziert wurde.

3. Elektronische Injektionsvorrichtung nach Anspruch 1, **dadurch gekennzeichnet, dass** ein Signal von einem Schalter, das anzeigt, dass der Injektionsknopf gedrückt wurde, als Anzeige dafür verwendet wird, dass eine eingestellte Dosis injiziert wurde.

4. Elektronische Injektionsvorrichtung nach einem der vorangehenden Ansprüche, **dadurch gekennzeichnet, dass** eine Positionsanzeige bereitgestellt ist, welche die Schaltung derart steuert, dass sie nur dann die Verabreichung einer Luftschussdosis durchführt, wenn die Positionsanzeige anzeigt, dass die Vorrichtung vertikal mit der Nadelspitze nach oben gehalten wird.

5. Elektronische Injektionsvorrichtung nach Anspruch 1, **dadurch gekennzeichnet, dass** die Dosiseinstellung elektromechanisch durch einen Elektromotor (3, 13) verwirklicht ist, der durch die elektronische Schaltung gesteuert wird, um den Injektionsknopf aus dem Gehäuse um einen Abstand je nach eingestellter Dosis anzuheben, und die Injektion manuell durch Zurückdrücken des Injektionsknopfs durchgeführt wird.

6. Elektronische Injektionsvorrichtung nach Anspruch 1, **dadurch gekennzeichnet, dass** die Betätigung des Injektionsknopfs bewirkt, dass ein Motor elek-tromechanisch eine Dosis aus der Ampulle drückt, wobei der Motor durch die elektronische Schaltung gesteuert wird, um eine automatisch eingestellte Luftschussdosis, wenn die Schaltung in ihrem Luftschussmodus arbeitet, und eine durch Betrieb des Dosiseinstellungsmittel eingestellte Dosis, wenn die Schaltung in ihrem Dosisinjektionsmodus arbeitet, auszudrücken.

7. Elektronische Injektionsvorrichtung nach einem der vorangehenden Ansprüche, **dadurch gekennzeichnet, dass** die Schaltung derart gestaltet ist, dass sie kein Signal vom Dosiseinstellungsmittel empfangen kann, bis mindestens eine Luftschussdosis verabreicht wurde.

8. Elektronische Injektionsvorrichtung nach einem der vorangehenden Ansprüche, **dadurch gekennzeichnet, dass** die Schaltung einen Speicher umfasst, worin zurück liegende Informationen von injizierten Dosen und die Zeit für ihre Injektion gespeichert sind.

9. Elektronische Injektionsvorrichtung nach einem der vorangehenden Ansprüche, **dadurch gekennzeichnet, dass** die Schaltung einen Speicher umfasst, wobei die Größe aller Luftschüsse und Injektionen zu der Größe des Gesamtinhalts einer neuen Ampulle summiert und davon subtrahiert werden, um den Speicher mit einer Information der Größe der übrigen Menge an Medikament in der Ampulle zurückzulassen.

10. Elektronische Injektionsvorrichtung nach einem der vorangehenden Ansprüche, **dadurch gekennzeichnet, dass** die derzeit eingestellte Dosis mit der übrigen Menge an Medikament in der Ampulle verglichen wird, um eine Blockierung für das Einstellen einer diese übrige Menge übersteigenden Dosis zu ermöglichen.

## Revendications

1. Dispositif électronique d'injection comprenant un boîtier (31), un moyen de réglage de dose (4, 5 ; 14, 15), un bouton d'injection (7, 17) et un circuit électronique (1, 11), ledit circuit électronique étant couplé audit moyen de réglage de dose et ledit circuit électronique étant adapté pour lire une taille de dose correspondant au fonctionnement du moyen de réglage de dose, une ampoule (32) contenant un médicament raccordée à une aiguille (35) montée à l'extrémité distale de l'ampoule, le dispositif étant adapté pour faire sortir par pression le médicament de l'ampoule au travers de l'aiguille lorsque le bouton d'injection est actionné, un afficheur (6, 16, 36) piloté par le circuit électronique et adapté pour montrer la dose réglée par le fonctionnement du moyen de réglage de dose,
**caractérisé en ce que** le circuit électronique est conçu pour fonctionner dans deux modes alternatifs:
un mode de purge d'air dans lequel il commande un préréglage automatique d'une dose de purge d'air destinée à être expulsée de l'ampoule au travers de l'aiguille lorsque le bouton d'injection est actionné,
et un mode d'injection de dose dans lequel une dose réglée par le fonctionnement du moyen de réglage de dose est injectée par le fonctionnement du bouton d'injection,
dans lequel ledit circuit se trouve dans le mode de purge d'air lorsque le dispositif est mis en service mais qu'aucune dose n'a été réglée,
ledit circuit passe vers le mode d'injection de dose lorsque le moyen de réglage de dose a été mis en fonctionnement,
et ledit circuit retourne dans le mode de purge d'air lorsque le circuit reçoit un signal indiquant qu'une dose réglée a été injectée.

2. Dispositif électronique d'injection selon la revendication 1, **caractérisé en ce qu'**un signal provenant d'un commutateur indiquant qu'un capuchon de protection (43) a été placé est considéré comme indiquant qu'une dose réglée a été injectée.

3. Dispositif électronique d'injection selon la revendication 1, **caractérisé en ce qu'**un signal provenant d'un commutateur indiquant que le bouton d'injection a été pressé est considéré comme indiquant qu'une dose réglée a été injectée.

4. Dispositif électronique d'injection selon l'une quelconque des revendications précédentes, **caractérisé en ce qu'**on prévoit un indicateur de position qui commande le circuit de façon à ce qu'il accomplisse uniquement ladite administration d'une dose de purge d'air lorsque l'indicateur de position indique que le dispositif est tenu verticalement avec l'aiguille pointée vers le haut.

5. Dispositif électronique d'injection selon la revendication 1, **caractérisé en ce que** le réglage de dose est réalisé de manière électromécanique au moyen d'un moteur électrique (3, 13) qui est commandé par le circuit électronique de façon à soulever le bouton d'injection du logement sur une distance correspondant à la dose réglée et **en ce qu'**on accomplit manuellement l'injection en ramenant par pression le bouton d'injection dans sa position initiale.

6. Dispositif électronique d'injection selon la revendication 1, **caractérisé en ce que** l'actionnement du bouton d'injection fait qu'un moteur fait sortir de manière électromécanique par pression une dose de l'ampoule, le moteur étant commandé par le circuit électronique pour faire sortir par pression une dose de purge d'air réglée automatiquement lorsque le circuit fonctionne dans son mode de purge d'air et une dose réglée par le fonctionnement du moyen de réglage de dose lorsque le circuit fonctionne dans son mode d'injection de dose.

7. Dispositif électronique d'injection selon l'une quelconque des revendications précédentes, **caractérisé en ce que** le circuit est conçu de sorte qu'il ne puisse pas recevoir un signal provenant du moyen de réglage de dose avant qu'au moins une dose de purge d'air n'ait été administrée.

8. Dispositif électronique d'injection selon l'une quelconque des revendications précédentes, **caractérisé en ce que** le circuit comprend une mémoire dans laquelle sont stockées des informations d'historique des doses injectées et du moment de leur injection.

9. Dispositif électronique d'injection selon l'une quelconque des revendications précédentes, **caractérisé en ce que** le circuit comprend une mémoire dans laquelle les tailles de toutes les purges d'air et injections sont additionnées et soustraites de la taille du contenu total d'une nouvelle ampoule pour laisser dans la mémoire les informations sur la taille de la quantité restante de médicaments dans l'ampoule.

10. Dispositif électronique d'injection selon l'une quelconque des revendications précédentes, **caractérisé en ce que** la dose réglée courante est comparée à la quantité de médicament restant dans l'ampoule pour permettre le blocage du réglage d'une dose dépassant cette quantité restante.
